# DEMANDE DE BREVET EUROPEEN

(11) **EP 1 380 603 A1**
(43) Date de publication de la demande: **14.01.2004**
(21) Numéro de dépôt: 03291519.1
(22) Date de dépôt: 20.06.2003
(51) Int. Cl.: C08F 220/16

(54) **Vernis à ongles**

(30) Priorité: 08.07.2002 FR 0208554
(71) Demandeur: L'OREAL, 75008 Paris (FR)
(72) Inventeur: Mougin, Nathalie, 75011 Paris (FR); Lion, Bertrand, 95270 Luzarches (FR); Vicic, Marco, 94360 Bry s/Marne (FR); Cazeneuve, Colette, 75007 Paris (FR)
(74) Mandataire: Boulard, Denis

(57) **Abrégé**

L'invention a pour objet un vernis à ongles comprenant, dans un milieu cosmétiquement acceptable, un polymère filmogène, le vernis étant apte à former un film ayant une adhérence, mesurée selon la norme ASTM D 3359-7, correspondant à un pourcentage de décollement inférieur à 35 %.

## Description

La présente invention a pour objet un vernis à ongles comprenant un polymère filmogène présentant de bonnes propriétés d'adhérence. L'invention a également pour objet un procédé de maquillage ou de soin des ongles.

La composition de vernis à ongles peut être employée comme base pour vernis, comme produit de maquillage des ongles, comme composition de finition, encore appelée "top-coat" en terminologie anglosaxonne, à appliquer sur le produit de maquillage des ongles ou bien encore comme produit de soin cosmétique des ongles. Ces compositions peuvent s'appliquer sur les ongles d'êtres humains ou bien encore sur des faux ongles.

Les compositions de vernis à ongles comprennent de façon connue un polymère filmogène dans un milieu solvant organique ou un milieu aqueux. Le vernis forme après séchage un film coloré ou incolore sur les ongles et permet ainsi d'embellir ou de protéger les ongles contre les agressions extérieurs tels que les frottements, les rayures. Or fréquemment, les vernis à ongles présentent une mauvaise tenue dans le temps : le film se détériore , notamment par écaillage ou décollement, au bout d'un ou deux jours. Une telle détérioration se produit souvent à l'extrémité de l'ongle. Lorsque le vernis est endommagé, l'utilisatrice doit alors retirer le vernis abimé puis procéder à une nouvelle application du vernis. L'utilisatrice peut également retoucher le vernis endommagé en appliquant partiellement du vernis mais ce type de retouche ne conduit pas à un maquillage parfaitement esthétique. Si l'utilisatrice ne fait rien, le vernis endommagé nuit à l'aspect estéthique du maquillage et n'assure pas une bonne protection de l'ongle.

D'autres vernis à ongles comme les vernis facilement pelable ou les vernis démaquillable à l'eau ne confèrent pas non plus une bonne tenue dans le temps.

Un besoin existe donc de disposer de vernis àongles permettant d'obtenir un film déposé sur les ongles présentant une tenue dans le temps satisfaisante, sans présenter de défaut inesthétique.

Le but de la présente invention est donc de proposer une composition de vernis à ongles présentant de bonnes propriétés de tenue dans le temps, en particulier une bonne résistance aux frottements, à l'eau, à l'écaillage.

Les inventeurs ont découvert qu'un vernis à ongles présentant une adhérence correspondant à un faible pourcentage de décollement permettait d'obtenir un vernis ayant une bonne tenue dans le temps.

De façon plus précise, l'invention a pour objet une composition de vernis à ongles comprenant, dans un milieu cosmétiquement acceptable, un polymère filmogène, le vernis étant apte à former un film ayant une adhérence, mesurée selon la norme ASTM D 3359-7, correspondant à un pourcentage de décollement inférieur à 35 %.

L'invention a aussi pour objet un procédé cosmétique de maquillage ou de soin non thérapeutique des ongles comprenant l'application sur les ongles d'au moins une couche de la composition de vernis à ongles telle que définie précédemment.

Dans la présente demande, on entend par "polymère filmogène", un polymère apte à former à lui seul, ou en présence d'agent auxiliaire de filmification, un film continu et adhérent sur l'ongle, à une température allant de 20 °C à 30 °C.

L'adhérence du film de vernis à ongles est déterminé à l'aide du test de quadrillage défini dans la norme ASTM D3359.

On dépose une couche de 50 µm d'épaisseur, après séchage pendant 24 heures à 23 ± 2 °C et à 55 ± 5 % d'humidité relative, sur une plaque de polyamide vendue sous la dénomination Nylon 6 par la société Goodfellow présentant une rugosité comprise entre 10 et 500 nm . Le film est découpé sous forme d'un quadrillage conformément à la norme ASTM D3359 à l'aide d'un peigne à 6 lames espacées chacunes de 1 mm. Le film est ainsi découpé dans tout son épaisseur. Puis on applique sur le film découpé un papier adhésif normalisé ISO 2409 de telle sorte que le papier adhésif soit en contact avec toute la surface du film découpé. Puis on retire le papier adhésif du film quadrillé et on détermine le nombre de carreau de film qui ont été détachés de la plaque par le papier adhésif.

On détermine alors le pourcentage de décollement du film comme décrit dans la norme ASTM D3359, pour une moyenne de 10 échantillons.

Le vernis selon l'invention présente un pourcentage de décollement inférieur à 35 %, de préférence inférieur à 15 %, et préférentiellement inférieur à 5 %.

Avantageusement, le polymère filmogène a une température de transition vitreuse (Tg) allant de -100 °C à 100 °C, de préférence allant de -50 °C à 80 °C, préférentiellement allant de 0 °C à +80 °C, et plus préférentiellement allant de +20 °C à +80 °C.

La mesure de la température de transition vitreuse (Tg) du polymère est effectuée par DMTA (Dynamical and Mechanical Température Analysis ou Analyse dynamique et mécanique de température).

Pour mesurer la température de transition vitreuse (Tg) du polymère, on effectue des essais de viscoélasticimétrie avec un appareil DMTA de Polymer TA Instruments (modèle DMA2980), sur un échantillon de film de polymère d'environ 150 ± 50 µm d'épaisseur, 5 mm de largeur et 10 mm de longueur, après séchage pendant 24 heures à 23 °C et 50-55 % d'humidité relative. On impose à cet échantillon une sollicitation de traction. L'échantillon subit une force statique de 0,01 N à laquelle se superpose un déplacement sinusoïdal de ± 8 µm à la fréquence de 1 Hz. On travaille ainsi dans le domaine linéaire, sous de faibles niveaux de déformation. Cette sollicitation de traction est effectuée sur l'échantillon à des températures variant de -150 °C à + 220 °C, avec une variation de température de 3 °C par minute.

On mesure alors le module complexe E* = E' + iE" du polymère testé en fonction de la température.

De ces mesures, on déduit les modules dynamiques E' de conservation et E" de perte, ainsi que le pouvoir amortissant : tgδ = E"/E'.

Puis on trace la courbe des valeurs de tgδ en fonction de la température ; cette courbe présente au moins un pic. La température de transition vitreuse Tg du polymère correspond à la température à laquelle se situe le sommet de ce pic.

Lorsque la courbe présente au moins 2 pics (dans ce cas, le polymère présente au moins 2 Tg), on prend comme valeur de Tg du polymère testé la température pour laquelle la courbe présente le pic de plus forte amplitude (c'est à dire correspondant à la plus grande valeur de tgδ ; on ne considère dans ce cas que la Tg « majoritaire » comme valeur de Tg du polymère testé).

Selon un mode de réalisation de l'invention, le vernis à ongles selon l'invention est apte à former un film ayant un pouvoir amortissant tgδ allant de 0,5 à 1,6, et de préférence allant de 0,8 à 1,4.

Avantageusement, le vernis à ongles selon l'invention est apte à former un film ayant un module de conservation E' inférieur à 300 MPa, de préférence inférieur à 100 MPa, et plus préférentiellement inférieur à 80 MPa.

Le module de conservation E' et le pouvoir amortissant sont déterminés selon le protocole d'essais de viscoélastimétrie décrit précédemment mais effectués à la température constante de 23 °C et l'échantillon subssant une force statique de 0,01 N à laquelle se superpose un déplacement sinusoïdal de ± 8 µm à la fréquence de 20 Hz.

Avantageusement, le polymère filmogène est insoluble dans l'eau à 25 °C, c'est-à-dire qu'il est soluble à moins de 1 % en poids dans l'eau à 25 °C (solubilité inféreiure à 1 % en poids). Le polymère est de préférence soluble dans les solvants organiques, comme l'acétate d'éthyle ou l'acétate de butyle (solubilité supérieure à 90 % en poids à 25 °C).

Le polymère filmogène a notamment un poids moléculaire moyen en nombre inférieur ou égal à 300000, notamment allant de 5000 à 300 000, et de préférence allant de 5000 à 150000.

Le polymère filmogène permettant d'obtenir les propriétés d'adhérence du vernis peut être choisi parmi les polymères radicalaires, les polycondensats. Le polymère est de préférence non réticulé.

En particulier, le polymère acrylique peut être un polymère comprenant :
- au moins un premier monomère choisi parmi les monomères à insaturation éthylénique comportant une fonction acide carboxylique ou sulfonique et/ou une fonction amide, en particulier choisi parmi l'acide (méth)acrylique, l'acide crotonique, l'acide acrylamido propane sulfonique, les (méth)acrylamides de N-alkyle ayant un groupe alkyle en C₁-C₁₂, de préférence en C₁-C₆; les monomères à insaturation éthylénique comportant au moins un atome de chlore comme le chlorostyrène ; les monomères à insaturation éthylénique comportant un groupe hydroxyle comme l'alcool vinylique.
- au moins un deuxième monomère choisi parmi les (méth)acrylate d'alkyle ayant un groupe alkyle en C₁-C₁₈, de préférence en C₁-C₆, les (méth)acrylate d'aryle ayant un groupe aryle en C₆-C₈, les (méth)acrylates de cycloalkyle ayant un groupe cycloalkyle en C₄-C₁₂.

Comme exemple de (méth)acrylamide de N-alkyle ayant un groupe alkyle en C₁-C₁₂, on peut citer l'acrylamide de N-tertiobutyle.

Comme exemple de (méth)acrylate d'alkyle ayant un groupe alkyle en C₁-C₁₂, on peut citer le (méth)acrylate de méthyle, le (méth)acrylate d'éthyle, le (méth)acrylate de n-propyle, le (méth)acrylate de n-butyle, le (méth)acrylate d'isobutyle.

Comme exemple de (méth)acrylate d'aryle ayant un aryle en C₆-C₈, on peut citer le (méth)acrylate de benzyle.

Comme exemple de (méth)acrylate de cycloalkyle ayant un groupe cycloalkyle en C₄-C₁₂, on peut citer le (méth)acrylate de cyclohexyle, le (méth)acrylate d'isobornyle.

Le polymère acrylique peut comprendre en outre un monomère additionnel choisi parmi les esters vinyliques, les monomères styréniques non chlorés et les monomères à insaturation éthylénique fluorés.

L'ester vinylique peut être par exemple l'acétate de vinyle, le néodécanoate de vinyle, le pivalate de vinyle, le benzoate de vinyle, le t-butyl benzoate de vinyle.

Comme monomères styréniques, on peut citer le styrène et l'alpha-méthyl styrène. Comme monomères à insaturation éthylénique fluorés, on peut citer le méthacrylate de trifluoroéthyle.

Avantageusement, le polymère acrylique est choisi parmi les copolymères d'acide acrylique et de (méth)acrylate d'alkyle ayant un groupe alkyle en C₁-C₄, et en particulier les copolymères acide acrylique/méthacrylate de méthyle/acrylate d'isobutyle et les copolymères acide acrylique/acrylate de t-butyle/acrylate d'isobutyle.

De préférence, le polymère acrylique comprend :
- de 0,5 % à 50% en poids de premier monomère tel que décrit précédemment, de préférence de 2 % à 30 % en poids et préférentiellement de 5 % à 20 % en poids, par rapport au poids total de monomères du polymère.
- de 40 % à 99 % en poids de deuxième monomère tel que décrit précédemment, de préférence de 50 % à 95 % en poids, et préférentiellement de 60 % à 90 % en poids, par rapport au poids total de monomères du polymère,
- et éventuellement le complément à 100 % en poids de monomère additionnel tel que décrit précédemment.

Le polymère peut être par ailleurs un polycondensat, et notamment choisi parmi polyuréthanes, les polyurées, les polyurée-uréthanes, et leurs mélanges.

Le polycondensat est de préférence choisi parmi ceux formés par polycondensation :
- d'au moins un diisocyanate choisi parmi les diisocyanates d'alkyle linéaire ou ramifié en C₁-C₁₂, de préférence en C₁-C₆, les diisocyanates de cycloalkyle en C₄-C₂₀ , les diisocyanates d'aryle en C₆-C₂₀ ;
- d'au moins un prépolymère comprenant au moins 2 fonctions comportant un hydrogène labile, notamment un diol ou une diamine primaire ou secondaire, ayant une masse moléculaire en nombre allant de 500 à 50000, de préférence allant de 1000 à 3000 et préférentiellement allant de 500 à 8000 ;
- d'au moins un coupleur comprenant deux fonctions comportant un hydrogène labile, notamment un diol ou une diamine primaire ou secondaire ou un aminoalcool, ayant une masse moléculaire inférieure 500, notamment supérieur ou égal à 50 et inférieur à 500, et de préférence supérieur ou égal à 75 et inférieur à 500.

Le diisocyanate peut être par exemple le diisocyanate d'hexaméthylène, le diisocyanate d'isophorone, le diisocyanate de dicyclohexylméthane, le diisocyanate de toluène, le diisocyanate de diphénylméthane, le diisocyanate de dicyclohexylméthane, le diisocyanate de tétraméthylxylylène.

Le prépolymère décrit précédemment peut être par exemple un diol de polyoxyde de tétramethylène ayant de 10 à 80 motifs d'oxyde de tétraméthylène; un polydiméthylsiloxane comprenant des groupements terminaux de type alkylène (C₂-C₈)amino alkyl (C₂-C₈) ou ou de type ω-hydroxyalkyle en C₂-C₈ ; un polybutadiène hydrogéné ayant des groupes hydroxyle terminaux.

Le prépolymère est préférentiellement non hydrosoluble (le prépolymère a une solubilité dans l'eau inférieure à 1 % en poids, à 25 °C).

Le coupleur peut être par exemple le butanediol, le néopentylglycol, l'aminoéthanol, le propylèneglycol, l'éthylèneglycol, le diéthylèneglycol, le triéthylèneglycol, le cyclohexane diméthanol.

Avantageusement, le prépolymère et le coupleur décrits précédemment sont présents dans le polyuréthane en une teneur telle que le rapport molaire prépolymère / coupleur va de 1 à 1/5 et le rapport molaire (prépolymère + coupleur) / diisocyanate va de 0,9 à 1,1.

De préférence, le polymère est tel que le prépolymère, le diisocyanate et le coupleur sont présents dans le polymère selon un rapport molaire :
Prépolymère : 1
Diisocyanate : 2 à 6
Coupleur : 1 à 5.

Lorsque le rapport pondéral (prépolymère + coupleur) / diisocyanate est inférieur à 1, les groupes isocyanates libres sont bloqués par réaction avec un composé comprenant un hydrogène labile comme l'éthanol.

Le polymère filmogène peut être présent dans la composition de vernis à ongles en une teneur allant de 0.1% à 60% en poids, par rapport au poids total de la composition, et de préférence allant de 0.1% à 40% en poids.

Le vernis à ongles selon l'invention peut comprendre en outre un polymère filmogène additionnel, appelée couramment résine, comme les résines sulfonamides, les résines alkyde, les esters de cellulose tel que l'acétobutyrate de cellulose, l'acétate de cellulose, l'acétopropionate de cellulose.

Le polymère filmogène additionnel peut être présent en une teneur allant de 0,01 % à 50 % en poids, par rapport au poids total de la composition, et de préférence allant de 1 % à 30 % en poids.

La composition de vernis à ongles selon l'invention peut comprendre un agent auxiliaire de filmification pour améliorer les propriétés filmogènes du vernis.

L'agent auxiliaire de filmification peut être choisi parmi tous les composés connus de l'homme du métier comme étant susceptibles de remplir la fonction recherchée, et être notamment choisi parmi les agents plastifiants.

Comme exemple d'agent plastifiant, on peut citer :
les citrates tels que le citrate de triéthyle, le citrate de tributyle, l'acétylcitrate de triéthyle, l'acétylcitrate de tributyle, l'acétylcitrate de triéthyl-2 hexyle
les phtalates tels que le phtalate de diéthyle, le phtalate de dibutyle, le phtalate de dioctyle, le phtalate de dipentyle, le phtalate de diméthoxyéthyle,
le phosphate de tricrésyle, le benzoate de benzyle, le phosphate de tributyle, l'acétylricinoléate de butyle, l'acétylricinoléate de glycéryle, le glycolate de butyle, le phosphate de tributoxyéthyle, le phosphate de triphényle, le tartrate de dibutyle, le camphre, le triacétate de glycérol, le N-éthyl-o,p-toluènesulfonamide, et leurs mélanges.

L'agent plastifiant peut être présent dans la composition en une teneur allant de 0,01 % à 10 % en poids, par rapport au poids total de la composition, et de préférence allant de 0,1 % à 5 % en poids. Avantageusement, le plastifiant est présent dans la composition selon un rapport pondéral polymère filmogène / plastifiant allant de 1,5 à 3.

La composition selon l'invention peut comprendre un milieu aqueux ou un milieu solvant organique, et de préférence un milieu solvant organique. Elle peut être notamment anhydre.

Le milieu aqueux de la composition peut être constitué essentiellement d'eau. La teneur en eau dans la composition peut aller de 10 % à 95 % en poids, par rapport au poids total de la composition, de préférence de 40 % à 90 % en poids, et mieux de 60 % à 85 % en poids.

La composition peut comprendre également un solvant miscible à l'eau comme les monoalcools inférieurs ayant de 1 à 5 atomes de carbone, les glycols ayant de 2 à 8 atomes de carbone, les cétones en C₃-C₄, les aldéhydes en C₂-C₄, notamment en une teneur pouvant aller de 0,1 % à 15 % en poids, par rapport au poids total de la composition.

Lorsque la composition comprend un milieu aqueux, le polymère filmogène est présent sous formes de particules solides dispersées dans le mileu aqueux. Une telle dispersion est connue sous le nom de latex ou de pseudolatex et peut être préparée selon les techniques bien connues de l'homme du métier.

Le milieu solvant organique de la composition peut comprendre un ou plusieurs solvants organiques choisis parmi :
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le n-propanol, le n-butanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, l'isododécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde
- leurs mélanges.

La teneur en solvant organique dans la composition peut aller de 10 % à 95 % en poids, par rapport au poids total de la composition, de préférence de 40 % à 90 % en poids, et mieux de 60 % à 85 % en poids.

La composition peut comprendre un agent épaississant, notamment pour conférer à la composition une consistance permettant une bonne application de la composition sur les ongles. L'agent épaississant est plus particulièrement un épaississant de solvant organique et peut être choisi parmi les silices hydrophobes, telles que celles dérites dans le document EP-A-898960, et par exemple commercialisées sous les références "AEROSIL R812®" par la société Degussa, "CAB-O-SIL TS-530®", "CAB-O-SIL TS-610®", "CAB-O-SIL TS-720®"par la société Cabot, "AEROSIL R972®", "AEROSIL R974®" par la société Degussa ; les argiles telles que la montmorillonite, l'hectorite stéralkonium, la bentonite stéralkonium ; les alkyléther de polysaccharides (notamment dont le groupe alkyle comporte de 1 à 24 atomes de carbones, de préférence de 1 à 10, mieux de 1 à 6, et plus spécialement de 1 à 3) tels que ceux décrits dans le document EP-A-898958, et par exemple commercialisés sous les dénominations "N-HANCE-AG 200®" et "N-HANCE AG 50®" par la société Aqualon.

L'agent épaississant peut être présent dans la composition selon l'invention en une teneur allant de 0,05 % à 10 % en poids, par rapport au poids total de la composition, et de préférence allant de 0,1 % à 3 % en poids.

La composition peut comprendre également une matière colorante qui peut être choisie parmi les colorants liposolubles, les pigments, les nacres, les paillettes.

Les colorants liposolubles peuvent être par exemple le rouge Soudan, le DC Red 17, le DC Green 6, le β-carotène, l'huile de soja, le brun Soudan, le DC Yellow 11, le DC Violet 2, le DC orange 5, le jaune quinoléine. Ils sont généralement présents dans la composition en une teneur pouvant aller de 0,01 % à 6 % en poids, par rapport au poids total de la composition, de préférence allant de 0,01 % à 3 % en poids.

Les pigments, les nacres et les pailettes peuvent être présents dans la composition, notamment la composition de base et/ou de surface, en une teneur allant de 0,01 % à 25 % en poids, par rapport au poids de la composition, de préférence de 0,01 % à 15 % en poids. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium, aluminium. Parmi les nacres, on peut citer le mica recouvert d'oxyde de titane, d'oxyde de fer, de pigment naturel ou d'oxychlorure de bismuth tel que le mica titane coloré.

La composition selon l'invention, notamment la composition de base et/ou de surface, peut en outre comprendre tout additif cosmétique connu de l'homme du métier comme étant susceptible d'être incorporé dans une telle composition, tels que les charges, les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants. Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.

L'invention est illustrée plus en détail dans les exemples suivants.

### Exemple 1 :

a) On a préparé le polymère filmogène suivant :
Copolymère méthacrylate de méthyle/acrylate d'isobutyle/acide acrylique (55/25/20 en poids)
   Dans un réacteur de 500ml équipé d'une agitation centrale, d'un réfrigérant, on introduit sous atmosphère d'azote 55 g de méthacrylate de méthyle, 25 g d'acrylate d'isobutyle, 20 g d'acide acrylique, 0,6 g d'amorceur bis(2-éthylhexanoylpéroxy)-2,5-diméthylhexane vendu sous la dénomination Trigonox 141 par la société AKZO CHEMIE , 70 g d'acétate de butyle et 30 g d'acétate d'éthyle.
   On agite à température ambiante puis on porte le milieu réactionnel à 90°C en 1
   heure. On laisse la réaction se développer à 90°C pendant 18 heures.
   On obtient au final le polymère dans le mélange acétate du butyle / acétate d'éthyle à une concentration de 50 % en poids. Le polymère présente les caractéristiques suivantes :
   Poids moléculaire moyen en nombre : 6500
   Température de transition vitreuse : + 65 °C
b) On a préparé un vernis à ongles ayant la composition suivante :
On dilue la solution de polymère obtenue dans un mélange acétate de butyle / acétate d'éthyle 70 / 30 en poids jusqu'à une concentration en poids de polymère égale à 25 %.
   La solution obtenue est ensuite appliquée sur les ongles. On obtient après séchage un vernis dont le film a les caractéristiques suivantes, mesurées selon les
   protocoles décrits précédemment dans la description :

Pourcentage de décollement : 30 %
Pouvoir amortissant tgδ : 0,8
module de conservation E' : 100 MPa

Le film présente une bonne tenue dans le temps, en particulier une bonne résistance à l'écaillage.

### Exemple 2 :

a) On a préparé le polymère filmogène suivant :
   Copolymère acrylate d'isobutyle/acrylate de tertio-butyle/acide acrylique (50/40/10 en poids)
   Dans un réacteur de 500ml équipé d'une agitation centrale, d'un réfrigérant, on introduit sous atmosphère d'azote 50 g d'acrylate d'isobutyle, 40 g d'acrylate de tertiobutyle, 10 g d'acide acrylique, 0,2 g d'amorceur bis(2-éthylhexanoylpéroxy)-2,5-diméthylhexane, 100 g d'éthanol.
   On agite à température ambiante puis on porte le milieu réactionnel à 77°C en laissant réagir pendant 10 heures.
   Le polymère en solution obtenu est précipité dans de l'éther de pétrole puis séché en étuve à 40°C sous vide en présence d'anhydride phosphorique.
   Le polymère présente les caractéristiques suivantes :
   Poids moléculaire moyen en nombre : 51500
   Température de transition vitreuse : + 40 °C
b) On a préparé un vernis à ongles ayant la composition suivante :
   On a dissout 25 g du polymère obtenu dans 70 g d'un mélange acétate de butyle / acétate d'éthyle 70 / 30 en poids. La solution obtenue est ensuite appliquée sur les ongles. On obtient après séchage un vernis dont le film a les caractéristiques suivantes, mesurées selon les
   protocoles décrits précédemment dans la description :
   Pourcentage de décollement : compris entre 5 et 15 %
   Pouvoir amortissant tgδ : 1,3
   module de conservation E' : 26 MPa
Le film présente une bonne tenue dans le temps, en particulier une bonne résistance à l'écaillage.

## Revendications

1. Vernis à ongles comprenant, dans un milieu cosmétiquement acceptable, un polymère filmogène, le vernis étant apte à former un film ayant une adhérence, mesurée selon la norme ASTM D 3359-7, correspondant à un pourcentage de décollement inférieur à 35 %.

2. Vernis à ongles selon la revendication 1, **caractérisé par le fait que** le pourcentage de décollement est inférieur à 15 %.

3. Vernis à ongles selon la revendication 1, **caractérisé par le fait que** le pourcentage de décollement est inférieur à 5 %.

4. Vernis à ongles selon l'une quelconque des revendications précédentes, **caractérisé par le fait que** le polymère à une température de transition vitreuse allant de -100 °C à +100 °C.

5. Vernis à ongles selon l'une quelconque des revendications précédentes, **caractérisé par le fait que** le polymère a une température de transition vitreuse allant de -50 °C à +80 °C, de préférence allant de 0 °C à + 80 °C, et préférentiellement allant de +20 °C à +80 °C.

6. Vernis à ongles selon l'une quelconque des revendications précédentes, **caractérisé par le fait qu'**il est apte à former un film ayant un pouvoir amortissant tgδ allant de 0,5 à 1,6.

7. Vernis à ongles selon la revendication 6, **caractérisé par le fait que** pouvoir amortisant du film va de 0,8 à 1,4.

8. Vernis à ongles selon l'une quelconque des revendications précédentes, **caractérisé par le fait qu'**il est apte à former un film ayant un module de conservation E' inférieur à 300 MPa.

9. Vernis à ongles selon la revendication 8, **caractérisé par le fait que** le film a un module de conservation inférieur à 150 MPa, et de préférence inférieur à 80 MPa.

10. Vernis à ongles selon l'une quelconque des revendications précédentes, **caractérisé par le fait que** le polymère filmogène est choisi parmi les polymères acryliques, les polyuréthanes, les polyurées, les polyurée-uréthanes.

11. Vernis à ongles selon l'une quelconque des revendications précédentes, **caractérisé par le fait que** le polymère filmogène est un polymère comprenant :
- au moins un premier monomère choisi parmi les monomères à insaturation éthylénique comportant une fonction acide carboxylique ou sulfonique et/ou une fonction amide ; les monomères à insaturation éthylénique comportant au moins un atome de chlore ; les monomères à insaturation éthylénique comportant un groupe hydroxyle.
- au moins un deuxième monomère choisi parmi les (méth)acrylates d'alkyle ayant un goupe alkyle en C₁-C₁₈, les (méth)acrylates d'aryle ayant un groupe aryle en C₆-C₈, les (méth)acrylates de cycloalkyle ayant un groupe cycloalkyle en C₄-C₈.

12. Vernis à ongles selon la revendication précédente, **caractérisé par le fait que** :
- un premier monomère est choisi parmi l'acide (méth)acrylique, l'acide crotonique, l'acide acrylamido propane sulfonique, les (méth)acrylamides de N-alkyle ayant un groupe alkyle en C₁-C₁₂, le chlorostyrène, l'alcool vinylique.

13. Vernis à ongles selon la revendication 11 ou 12, **caractérisé par le fait que** le premier monomère est choisi parmi l'acide (méth)acrylique, l'acide crotonique, l'acide (méth)acrylique, l'acide crotonique, l'acide acrylamido propane sulfonique, l'acrylamide de N-tertiobutyle, le chlorostyrène, l'alcool vinylique.

14. Vernis à ongles selon l'une quelconque des revendications 11 à 13, **caractérisé par le fait que** le deuxième monomère est choisi parmi le (méth)acrylate de méthyle, le (méth)acrylate d'éthyle, le (méth)acrylate de n-propyle, le (méth)acrylate de n-butyle, le (méth)acrylate d'isobutyle, le (méth)acrylate de cyclohexyle, le (méth)acrylate d'isobornyle.

15. Vernis à ongles selon la revendication 11 ou 12, **caractérisé par le fait que** le polymère acrylique comprend un monomère additionnel choisi parmi les esters vinyliques, les monomères styréniques non chlorés et les monomères à insaturation éthylénique fluorés.

16. Vernis à ongles selon la revendication 15, **caractérisé par le fait que** le monomère additionnel est choisi parmi choisi parmi l'acétate de vinyle, le néodécanoate de vinyle, le pivalate de vinyle, le benzoate de vinyle, le t-butyl benzoate de vinyle, le styrène, l'alpha-méthyl styrène, le méthacrylate de trifluoroéthyle.

17. Vernis à ongles selon l'une quelconque des revendications 11 à 16, **caractérisé par le fait que** le polymère acrylique comprend :
- de 0,5 % à 50 % en poids de premier monomère,
- de 40 % à 99 % en poids de deuxième monomère,
- et éventuellement le complément à 100 % de monomère additionnel.

18. Vernis à ongles selon l'une quelconque des revendications 11 à 17, **caractérisé par le fait que** le polymère acrylique comprend :
- de 2 % à 30 % en poids de premier monomère,
- de 50 % à 95 % en poids de deuxième monomère ;
- et éventuellement le complément à 100 % de monomère additionnel.

19. Vernis à ongles selon l'une quelconque des revendications 11 à 18, **caractérisé par le fait que** le polymère acrylique comprend :
- de 5 % à 20 % en poids de premier monomère,
- de 60 % à 90 % en poids de deuxième monomère ; .
et éventuellement le complément à 100 % de monomère additionnel

20. Vernis à ongles selon l'une quelconque des revendications 11 à 17, **caractérisé par le fait que** le polymère acrylique est choisi parmi les copolymères d'acide acrylique et/ou d'acrylamide de tertiobutyle et de (méth)acrylate d'alkyle ayant un groupe alkyle en C₁-C₄.

21. Vernis à ongles selon l'une quelconque des revendications précédentes, **caractérisé par le fait que** le polymère filmogène est choisi parmi les copolymères de d'acide acrylique et de (méth)acrylate d'alkyle ayant un groupe alkyle en C₁-C₄.

22. Vernis à ongles selon l'une quelconque des revendications 11 à 18, **caractérisé par le fait que** le polymère acrylique est choisi parmi les copolymères acide acrylique/méthacrylate de méthyle/acrylate d'isobutyle et les copolymères acide acrylique/acrylate de t-butyle/acrylate d'isobutyle.

23. Vernis à ongles selon l'une quelconque des revendications précédentes, **caractérisé par le fait que** le polymère filmogène est choisi parmi les polyuréthanes, les polyurées, les polyurée-uréthanes, et leurs mélanges.

24. Vernis à ongles selon l'une quelconque des revendications 1 à 10, **caractérisé par le fait que** le polymère filmogène est un polycondensat formé par polycondensation :
- d'au moins un diisocyanate choisi parmi les diisocyanates d'alkyle linéaire ou ramifié en C₁-C₁₂, les diisocyanates de cycloalkyle en C₄-C₂₀ , les diisocyanates d'aryle en C₆-C₂₀;
- d'au moins un prépolymère comprenant au moins 2 fonctions comportant un hydrogène labile, notamment un diol ou une diamine primaire ou secondaire, ayant une masse moléculaire en nombre allant de 500 à 50000, de préférence allant de 1000 à 3000 et préférentiellement allant de 500 à 8000 ;
- d'au moins un coupleur comprenant deux fonctions comportant un hydrogène labile, notamment un diol ou une diamine primaire ou secondaire ou un aminoalcool, ayant une masse moléculaire inférieure 500, notamment supérieur ou égal à 50 et inférieur à 500, et de préférence supérieur ou égal à 75 et inférieur à 500.

25. Vernis à ongles selon la revendication précédente, **caractérisé par le fait que** le diisocyanate est choisi parmi le diisocyanate d'hexaméthylène, le diisocyanate d'isophorone, le diisocyanate de dicyclohexylméthane, le diisocyanate de toluène, le diisocyanate de diphénylméthane, le diisocyanate de dicyclohexylméthane, le diisocyanate de tétraméthylxylylène.

26. Vernis à ongles selon la revendication 24 ou 25, **caractérisé par le fait que** le prépolymère est choisi parmi un diol de polyoxyde de tétramethylène ayant de 10 à 80 motifs d'oxyde de tétraméthylène ; un polydiméthylsiloxane comprenant des groupements terminaux de type alkylène (C₂-C₈)amino alkyl (C₂-C₈) ou ou de type ω-hydroxyalkyle en C₂-C₈ ; un polybutadiène hydrogéné ayant des groupes hydroxyle terminaux.

27. Vernis à ongles selon l'une quelconque des revendications 24 à 26, **caractérisé par le fait que** le prépolymère est non hydrosoluble.

28. Vernis à ongles selon l'une quelconque des revendications 24 à 27, **caractérisé par le fait que** le coupleur est choisi parmi le butanediol, le néopentylglycol, l'aminoéthanol, le propylèneglycol, l'éthylèneglycol, le diéthylèneglycol, le triéthylèneglycol, le cyclohexane diméthanol.

29. Vernis à ongles selon l'une quelconque des revendications 24 à 28, **caractérisé par le fait que** le prépolymère et le coupleur sont présents dans le polyuréthane en une teneur telle que le rapport molaire prépolymère / coupleur va de 1 à 1/5 et le rapport molaire (prépolymère + coupleur) / diisocyanate va de 0,9 à 1,1.

30. Vernis à ongles selon l'une quelconque des revendications 24 à 29, **caractérisé par le fait que** le polymère est tel que le prépolymère, le diisocyanate et le coupleur sont présents dans le polymère selon un rapport molaire :
Prépolymère : 1
Diisocyanate : 2 à 6
Coupleur : 1 à 5.

31. Vernis à ongles selon l'une quelconque des revendications 24 à 29, **caractérisé par le fait que** lorsque le rapport pondéral (prépolymère + coupleur) / diisocyanate est inférieur à 1, les groupes isocyanates libres sont bloqués par réaction avec un composé comprenant un hydrogène labile comme l'éthanol.

32. Vernis à ongles selon l'une quelconque des revendications précédentes, **caractérisé par le fait que** le polymère filmogène a un poids moléculaire moyen en nombre inférieur ou égal à 300000, et de préférence allant de 10000 à 150000.

33. Vernis à ongles selon l'une quelconque des revendications précédentes, **caractérisé par le fait que** le polymère filmogène est présent en une teneur allant de 0,1 % à 60% en poids, par rapport au poids total de la composition, et de préférence allant de 0.1% à 40% en poids.

34. Vernis à ongles selon l'une quelconque des revendications précédentes, **caractérisé par le fait qu'**il comprend un polymère filmogène additionnel choisi parmi les résines sulfonamides, les résines alkyde, les esters de cellulose.

35. Vernis à ongles selon l'une quelconque des revendications précédentes, **caractérisé par le fait qu'**il comprend un agent plastifiant.

36. Vernis à ongles selon l'une quelconque des revendications précédentes, **caractérisé par le fait qu'**il comprend un milieu solvant organique.

37. Vernis à ongles selon la revendication précédente, **caractérisé par le fait qu'**il est anhydre.

38. Vernis à ongles selon l'une quelconque des revendications 1 à 37, **caractérisé par le fait qu'**il comprend un milieu aqueux.

39. Vernis à ongles selon l'une quelconque des revendications précédentes, **caractérisé par le fait qu'**il comprend un additif cosmétique choisi parmi les agents épaississants, les matières colorantes, les charges, les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants.

40. Procédé cosmétique de maquillage ou de soin non thérapeutique des ongles comprenant l'application sur les ongles d'au moins une couche de vernis à ongles selon l'une quelconque des revendications précédentes.
